(19) Europäisches Patentamt
European Patent Office
Office européen des brevets

(11) EP 4 679 079 A1

(12) EUROPEAN PATENT APPLICATION
published in accordance with Art. 153(4) EPC

(43) Date of publication:
**14.01.2026 Bulletin 2026/03**

(21) Application number: **24826248.7**

(22) Date of filing: **19.06.2024**

(51) International Patent Classification (IPC):
***G01N 27/623** (2021.01)* ***H01J 49/04** (2006.01)*
***H01J 49/16** (2006.01)* ***H01J 49/40** (2006.01)*

(86) International application number:
**PCT/KR2024/008466**

(87) International publication number:
**WO 2024/262932 (26.12.2024 Gazette 2024/52)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **19.06.2023 KR 20230078348**

(71) Applicant: **LG Chem, Ltd.**
**Seoul 07336 (KR)**

(72) Inventors:
- **YOU, Hyun Sik**
  **Daejeon 34122 (KR)**
- **BAE, Yongjin**
  **Daejeon 34122 (KR)**
- **KIM, Jo Il**
  **Daejeon 34122 (KR)**
- **LIM, Young Hee**
  **Daejeon 34122 (KR)**
- **WON, Young Chul**
  **Daejeon 34122 (KR)**
- **OH, Jeongmin**
  **Daejeon 34122 (KR)**
- **KIM, Jeonghyeon**
  **Daejeon 34122 (KR)**
- **KIM, Seung Tae**
  **Daejeon 34122 (KR)**

(74) Representative: **Plasseraud IP**
**104 Rue de Richelieu**
**CS92104**
**75080 Paris Cedex 02 (FR)**

(54) **METHOD FOR ANALYSING DEUTERIUM SUBSTITUTION RATES AND METHOD FOR PREDICTING DEUTERIUM SUBSTITUTION RATES**

(57) The present disclosure relates to a method for analyzing and predicting deuterium substitution rates of a deuterium-substituted sample using a mass spectrometry spectrum of the deuterium-substituted sample.

*FIG. 2*

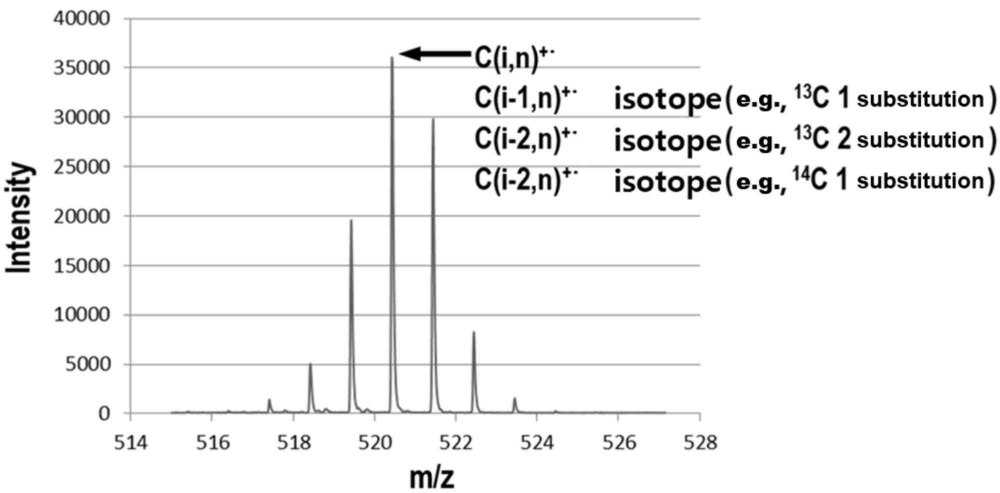


EP 4 679 079 A1

## Description

[TECHNICAL FIELD]

[Cross-Reference to Related Applications]

This application claims the benefit of priority based on Korean Patent Application No. 10-2023-0078348 filed on June 19, 2023, and all contents disclosed in the relevant Korean patent application are included as part of this specification.

The present disclosure relates to a method for analyzing and predicting deuterium substitution rates of a deuterium-substituted sample using a mass spectrum of the deuterium-substituted sample.

[BACKGROUND]

Hydrogen is the most abundant element in the universe, and is found in many compounds on earth, including water.

1-Hydrogen ($^1H$) and deuterium ($^2H$) are isotopes of hydrogen. The nucleus of 1-hydrogen consists of only one proton, while the nucleus of deuterium consists of one proton and one neutron.

Due to this difference in the number of neutrons, the atomic weights of 1-hydrogen and deuterium shows a weight difference of 1 Dalton, and similarly, even in the compound molecule in which 1-hydrogen is substituted by deuterium, the molecular weight of the compound shows a weight difference of 1 Dalton according to the number of substitutions.

This difference in atomic weight can lead to differences in physical and chemical characteristics, such as higher boiling point and melting point for deuterium compared to 1-hydrogen. Compounds in which deuterium is substituted at the hydrogen position in the compound also show chemical and physical characteristics similar to those of compounds in which a general hydrogen ($^1H$) is bonded, but may show some different behaviors due to the isotope effect.

By use of these chemical and physical characteristics, the presence or absence of existence of the materials, reactions, and pathways can be traced through the deuterium labeling method (D-labeling) in which hydrogen atoms in a molecule are replaced with deuterium atoms, and therefore, deuterium is widely used as an analytical tracer for functional materials in various fields such as agriculture, medicine, and natural science.

In particular, in organic light emitting diode(OLED) materials that have frequently been used in recent years, when 1-hydrogen at a specific position is substituted with deuterium, it exhibits superior effects in terms of luminescence efficiency and lifespan compared to 1-hydrogen isotopes. Therefore, research is underway to replace the 1-hydrogen position of luminescent materials with deuterium.

However, as mentioned above, since a compound containing only 1-hydrogen (hereinafter, 1-hydrogen compound) and a compound in which the hydrogen is substituted by deuterium (hereinafter, deuterated compound) are very similar in terms of physical and chemical characteristics other than molecular weight. Therefore, when 1-hydrogen compounds and deuterated compounds, especially compounds in which the hydrogen is substituted at different positions or with different numbers of deuterium substitutions, are mixed, it is very difficult to find out the substitution rates, etc. through analysis.

Therefore, studies are needed to develop methods for accurately confirming the substitution rates, etc. in a sample containing a deuterated compound.

[DETAILED DESCRIPTION OF THE INVENTION]

[Technical Problem]

It is an object of the present disclosure to provide an analytical method that can accurately confirm deuterium substitution rates in a sample containing a deuterated compound.

It is another object of the present disclosure to provide a method that can predict deuterium substitution rates of the product during the manufacturing process of deuterated compounds.

[Technical Solution]

Provided herein is a method for analyzing deuterium substitution rates of a sample material, comprising: a step 1 of obtaining a mass spectrum for a sample of an analysis object material including one or more compounds selected from the set of chemical species represented by {C(0,n), C(1,n), C(2,n), C(3,n), ..., C(i,n), C(i+1,n), ..., C(n-1,n), C(n,n)}; a step 2 of expressing the mass spectrum of the sample material as a linear combination of the mass spectra of each of element compound belonging to the set; and a step 3 of calculating the relative content value of each element compound belonging

to the set from the coefficients of the linear combination.

In the compound C(i,n) as each element constituting the set, C means that the compounds as each element constituting the set are identical compounds except for whether they are substituted by hydrogen or deuterium, n means the total number of hydrogen and deuterium bond sites in the compound C molecule, i means the number of deuterium substitutions in the element compound included in the set, and C(i,n) means a compound in which i of the total n hydrogen and deuterium bond sites are substituted by deuterium.

The set {C(0,n), C(1,n), C(2,n), C(3,n), ..., C(i,n), C(i+1,n), ..., C(n-1,n), C(n,n)} can also be expressed in other ways as follows. {C(i,n)|C(i,n) is a compound where i = 0 ~ n}; in the compound C(i,n) as each element constituting the set, C means that the compounds as each element constituting the set are identical to each other except for whether they are substituted by hydrogen or deuterium, n means the total number of hydrogen and deuterium bond sites in the compound C molecule, i means the number of deuterium substitutions in the element chemical species included in the set, and C(i,n) means a compound in which i of the total n hydrogen and deuterium bond sites is substituted by deuterium.

In some cases, the sample material to be analyzed may contain only pure materials in which the numbers of deuterium substitutions or the substitution positions are identical to each other, or may be in a mixture state, which is a mixed state of a group of compounds in which the numbers of deuterium substitutions or the substitution positions are different from each other.

According to an embodiment, the step 1 may be a step of obtaining a mass spectrum by MALDI-TOF MS measurement using an inorganic oxide matrix.

According to an embodiment, the inorganic oxide matrix may be an inorganic oxide matrix including at least one element selected from the group consisting of silicon, zirconium, aluminum, cerium, and the like.

According to an embodiment, the step 2 may eliminate the effect due to isotope using the natural abundance ratio of isotopes.

According to an embodiment, the step 2 may express the mass spectrum of the sample material as a linear combination of the mass spectra of each of the element compounds belonging to the set using least square approximation.

According to an embodiment, the method for analyzing deuterium substitution rates of the sample material may further comprise a step 4 of calculating an average deuterium substitution rate of a sample material from the relative content values of each of the element compounds belonging to the set.

According to an embodiment, the step 4 may calculate the average deuterium substitution rate of the sample material according to the following Mathematical Equation 1:

[Mathematical Equation 1]

$$SD(\%) = \frac{\sum_{i=0}^{n}(i \times P_i)}{n};$$

in Mathematical Equation 1, SD(%) is the average deuterium substitution rate of the sample material to be analyzed, n means the total number of hydrogen and deuterium bond sites in the compound C molecule, and $P_i$ is the relative amount(%) of a compound C(i,n) in which i of the total n hydrogen and deuterium bond sites are substituted with deuterium.

According to an embodiment, the $P_i$ may be calculated according to the following Mathematical Equation 2.

[Mathematical Equation 2]

$$P_i = \frac{a_i}{\sum_{i=0}^{n} a_i} \times 100$$

in Mathematical Equation 2, $P_i$ is the relative amount(%) of a compound C(i,n) in which i of the total n hydrogen and deuterium bond sites are substituted with deuterium, and $a_i$ is the weight of the C(i,n) molecule.

Also provided herein is a method for predicting deuterium substitution rates of the reaction product, comprising: a step of measuring an average deuterium substitution rate of a first reactant by the above-mentioned method, a step of measuring an average deuterium substitution rate of a second reactant by the above-mentioned method, and a step of predicting a deuterium substitution rate of a reaction product obtained by the reaction between the first reactant and the second reactant using the average deuterium substitution rate of the first reactant and the average deuterium substitution rate of the second reactant.

As used herein, the terms "a first," "a second," etc. are used herein to explain various constitutional elements, and the terms are only used to distinguish one constitutional element from the other constitutional elements.

The technical terms used herein is only to explain exemplary embodiments and is not intended to limit the scope of the present disclosure.

The singular forms "a," "an" and "the" are intended to include plural forms, unless the context clearly indicates otherwise.

It should be understood that the terms "comprise," "include", "have", etc. are used herein to specify the presence of stated features, integers, steps, components or combinations thereof, but do not preclude the presence or addition of one or more other features, integers, steps, components, or combinations thereof.

Although the present disclosure may have various forms and various modifications may be made thereto, specific examples will be exemplified and explained in detail below. However, it is not intended to limit the present disclosure to specific disclosure, and it should be understood that the present disclosure includes all the modifications, equivalents or replacements thereof without departing from the spirit and technical scope of the present disclosure.

Hereinafter, the present disclosure will be described in detail.

According to an aspect of the present disclosure, there is provided a method for analyzing deuterium substitution rates of a sample material, comprising: a step 1 of obtaining a mass spectrum for a sample of an analysis object material including one or more compounds selected from the set of chemical species represented by {C(0,n), C(1,n), C(2,n), C(3,n), ..., C(i,n), C(i+1,n), ..., C(n-1,n), C(n,n)}; a step 2 of expressing the mass spectrum of the sample material as a linear combination of the mass spectra of each of element compound belonging to the set; and a step 3 of calculating the relative content value of each element compound belonging to the set from the coefficients of the linear combination.

In the compound C(i,n) as each element constituting the set, C means that the compounds as each element constituting the set are identical compounds except for whether they are substituted by hydrogen or deuterium, n means the total number of hydrogen and deuterium bond sites in the compound C molecule, i means the number of deuterium substitutions in the element compound included in the set, and C(i,n) means a compound in which i of the total n hydrogen and deuterium bond sites are substituted by deuterium.

The set {C(0,n), C(1,n), C(2,n), C(3,n), ..., C(i,n), C(i+1,n), ..., C(n-1,n), C(n,n)} can also be expressed in other ways as follows. {C(i,n)|C(i,n) is a compound where i = 0 ~ n}; in the compound C(i,n) as each element constituting the set, C means that the compounds as each element constituting the set are identical to each other except for whether they are substituted by hydrogen or deuterium, n means the total number of hydrogen and deuterium bond sites in the compound C molecule, i means the number of deuterium substitutions in the element chemical species included in the set, and C(i,n) means a compound in which i of the total n hydrogen and deuterium bond sites is substituted by deuterium.

In a compound C in which the remaining element constitution and the interatomic bond relationship are identical except for whether they are substituted with hydrogen or deuterium, when there are n sites where hydrogen or deuterium is bonded, a compound having a structure in which no deuterium is substituted at al1, i.e., a 1-hydrogen compound whose deuterium substitution number is 0, can be represented by C(0, n). And, a compound having a structure in which one of n substitution sites is substituted by deuterium instead of hydrogen, i.e., a deuterated compound whose deuterium substitution number is 1, can be represented by C(1,n), a compound in which i of the n substitution sites are substituted by deuterium instead of hydrogen, i.e., a deuterated compound whose deuterium substitution number is i, can be represented by C(i,n), and a compound having a structure in which all n sites of the n substitution sites are substituted by deuterium instead of hydrogen, i.e., a deuterated compound whose deuterium substitution number is n, can be represented by C(n,n).

Here, the sample material to be analyzed may include one or more chemical species selected from the set of chemical species represented by {C(0,n), C(1,n), C(2,n), C(3,n), ... , C(i,n), C(i+1,n), ... , C(n-1,n), C(n,n)}.

That is, the sample material to be analyzed may include only pure materials in which the numbers of deuterium substitutions or the substitution positions are exactly identical, or may be in a mixed state of a group of compounds in which the numbers of deuterium substitutions or the substitution positions are different from each other.

Provided herein is a method for finding out average deuterium substitution rates of the sample material to be analyzed, which is a pure material or a mixture, that is, the ratio of the number of deuterium atoms to the total number of hydrogen or deuterium atoms existing in the sample material to be analyzed, through mass spectrum analysis.

First, a mass spectrum for a sample of an analysis object material including one or more compounds selected from the set of chemical species represented by {C(0,n), C(1,n), C(2,n), C(3,n), ..., C(i,n), C(i+1,n), ..., C(n-1,n), C(n,n)} is obtained.

According to an embodiment, the mass spectrum can be obtained by MALDI-TOF MS measurement using a spectral inorganic oxide matrix.

The inorganic oxide matrix may be an inorganic oxide matrix including at least one element selected from the group consisting of silicon, zirconium, aluminum, and cerium. More specifically, the inorganic oxide may be silica, zirconia, alumina, ceria, or an inorganic oxide in a state in which other silicon, zirconium, aluminum, and/or cerium atoms are mixed into the crystal lattice thereof, but the present disclosure is not necessarily limited thereto.

When the number of deuterium substitutions increases by one, the mass number of the molecule also increases by one. Since the above-mentioned inorganic oxide matrix does not generate a signal due to the material itself in MALDI-TOF MS analysis, and can generate a single ion species for the sample material to be analyzed, it is possible to eliminate the effect of increasing the mass number by one appeared by further bonding of another hydrogen to the molecule during MS analysis, and it can make it possible to exhibit only the effect of increasing the mass number by 1 due to deuterium substitution or the effect of increasing the mass number due to an isotope of an element other than deuterium that constitutes the compound (e.g., carbon, etc.).

FIGS. 1 and 2 are examples of typical mass spectra obtained by MALDI-TOF MS analysis.

FIG. 1 is assumed to be a spectrum obtained by MALDI-TOF MS analysis of a sample containing the above-mentioned compound C(i,n).

In FIG. 1, the highest peak in the center is the parent peak by C(i,n) that does not contain isotopes of elements other than deuterium (e.g., $^{13}C$, $^{14}C$, etc.), and the molecular weight at this point is defined as M0. However, in fact, the parent peak M0 of FIG. 1 is not a peak that appears by a single chemical species, but appears by a combination of peaks of several chemical species that have the same mass number and the same charge.

For example, the parent peak corresponding to the M0 appears by the total sum of the contributing parts described below.

i) the portion contributed by the radical ion $C(i,n)^{+\cdot}$, which is formed by losing one electron from the compound C(i,n);
ii) in the radical ion C(i-1,n)+ · (mass number: M0-1) which is formed by losing one electron from the compound C(i-1,n) (mass number: M0-1) having one fewer number of deuterium substitutions than the compound C(i,n), the portion contributed by the compound (mass number: M0) existing as an isotope (e.g., $^{13}C$) of the elements contained therein;
iii) the portion contributed by the compound $C(i-1,n)+H^+$ (mass number: M0) in which another hydrogen atom is further bonded to the radical ion $C(i-1,n)^{+\cdot}$ which is formed by losing one electron from the compound C(i-1,n) (mass number: M0-1) having one fewer number of deuterium substitutions than the compound C(i,n);
iv) the portion contributed by a compound (mass number: M0) in which a compound C(i-2,n) (mass number: M0-2) having two fewer number of deuterium substitutions than the compound C(i,n) loses one electron to form a radical ion $C(i-2,n)^{+\cdot}$, to which another hydrogen atom is further bonded (mass number: M0-1), and one of the elements contained therein exists as an isotope with mass number +1 (e.g., one of the carbon atoms is $^{13}C$);
v) in the radical ion $C(i-2,n)^{+\cdot}$ (mass number: M0-2) which is formed by losing one electron from the compound C(i-2,n) (mass number: M0-2) having two fewer numbers of deuterium substitutions than the compound C(i,n), the portion contributed by the compound (mass number: M0) in which one of the elements contained therein exists as an isotope with mass number +2 (e.g., one carbon atom is $^{14}C$);
vi) in the radical ion $C(i-2,n)^{+\cdot}$ (mass number: M0-2) which is formed by losing one electron from the compound C(i-2,n) (mass number: M0-2) having two fewer numbers of deuterium substitutions than the compound C(i,n), the portion contributed by the compound (mass number: M0) in which two of the elements contained therein exist as isotopes with mass number +1 (e.g., two carbon atoms are $^{13}C$)... .

By the same logic as above, another hydrogen is further bonded during the MS measurement process, or the mass number may increase or decrease due to the effect due to isotopes (e.g., $^{13}C$, $^{14}C$) of the elements included therein existing in the molecule, and among these, a chemical species with the same charge-to-mass ratio is detected as one peak. This is also applicable to other peaks besides the parent peak.

However, according to one embodiment, when MALDI-TOF MS measurement is conducted using an inorganic oxide matrix, it is possible to eliminate the case where hydrogen is further bonded and the mass number increases by 1, and it can make it possible to exhibit only the effects due to deuterium substitution and isotopes of elements other than deuterium (e.g., carbon, etc.).

FIG. 2 is assumed to be a spectrum obtained by performing MALDI-TOF MS analysis of a sample containing the above-mentioned compound C(i,n) using an inorganic oxide matrix.

In this case, the parent peak in FIG. 2 is appeared by the sum of the contributing portions mentioned below.

i) the portion contributed by the radical ion $C(i,n)^+$ , which is formed by losing one electron from the compound C(i,n);
ii) in the radical ion $C(i-1,n)^{+\cdot}$ (mass number: M0-2) which is formed by losing one electron from the compound C(i-1,n) (mass number: M0-1) having one fewer number of deuterium substitution than the compound C(i,n), the portion contributed by the compound (mass number: M0) in which one of the elements contained therein exists as an isotope with mass number +1 (e.g., one of the carbon atoms is $^{13}C$);
iii) in the radical ion $C(i-2,n)^{+\cdot}$ (mass number: M0-2) which is formed by losing one electron from the compound C(i-2,n) (mass number: M0-2) having two fewer number of deuterium substitutions than the compound C(i,n), the portion contributed by the compound (mass number: M0) in which two of the elements contained therein exist as isotopes with mass number +1 (e.g., two of the carbon atoms are $^{13}C$);
iv) In the radical ion $C(i-2,n)^{+\cdot}$ (mass number: M0-2) which is formed by losing one electron from the compound C(i-2,n) (mass number: M0-2) having two fewer numbers of deuterium substitutions than the compound C(i,n), the

portion contributed by the compound (mass number: M0) in which one of the elements contained therein exists as an isotope with mass number +2 (e.g., one of the carbon atoms is $^{14}C$) ... .

By the same logic as above, the mass number may increase or decrease by one due to the effect caused by the number of deuterium substitutions or the isotopes of the elements existing in the molecule (e.g., isotopes of carbon atoms such as $^{13}C$, $^{14}C$), and among these, a chemical species with the same charge-to-mass ratio is detected as one peak. This is also applicable to other peaks besides the parent peak.

Therefore, if the effect due to isotope (e.g., carbon isotope) can be eliminated, only the effect caused by the number of deuterium substitutions can remain.

According to an embodiment, in the step 2, the mass increase effect caused by isotopes other than deuterium can be eliminated by using the natural abundance ratio of isotopes, specifically elements constituting the compound (e.g., carbon, etc.). That is, in the sample material to be analyzed in the present disclosure, some or all of the 1-hydrogen of a specific compound is intentionally substituted by deuterium, and the deuterium abundance ratio in the sample material to be analyzed may be different from the natural abundance ratio of deuterium.

However, in the sample material to be analyzed in the present disclosure, isotopes of other constituent elements except for deuterium, specifically, for example, elements such as carbon, oxygen, and nitrogen, exist according to the natural abundance ratio of the corresponding isotopes, unless there are special circumstances.

For example, it is known that the natural abundance ratio of carbon ($^{12}C$) with a mass number of 12 is about 98.9%; the natural abundance ratio of carbon ($^{13}C$) with a mass number of 13 is about 1.1%; and the natural abundance ratio of carbon ($^{14}C$) with a mass number of 14 is about 0.0000000001%. Therefore, by using this natural abundances ratio, the effect due to carbon isotopes can be eliminated from the resulting mass spectrum.

In another example, since it is known that the natural abundance ratio of oxygen ($^{16}O$) with a mass number of 16 is about 99.757%; the natural abundance ratio of oxygen ($^{17}O$) with a mass number of 17 is about $3.8\times10^{-4}$; and the natural abundance ratio of oxygen ($^{18}O$) with a mass number of 18 is about $2.05\times10^{-3}$. By using this natural abundance ratio, the effect due to oxygen isotopes can be eliminated from the resulting mass spectrum.

In another embodiment, it is known that the natural abundance ratio of nitrogen ($^{14}N$) with a mass number of 14 is about 99.636%; the natural abundance ration of nitrogen ($^{15}N$) with a mass number of 15 is about 0.364%. Therefore, by using this natural abundance ratio, the effect due to nitrogen isotopes can be eliminated from the resulting mass spectrum.

In addition to the elements mentioned above, if an isotope exists in an element contained in a compound, it is possible to eliminate the effects due to the isotopes, i.e., the effect due to mass number change, by using the natural abundance ratio of the isotopes.

According to an embodiment, in the step 2, the mass spectrum of the sample material can be expressed as a linear combination of the mass spectrum of each element chemical species belonging to the set using least square approximation.

That is, each peak that appears in the mass spectrum obtained by analyzing the entire sample material to be analyzed is separated into a linear combination of the mass spectrum of each element chemical species included in the material to be analyzed, which can be considered to be a type of deconvolution.

FIG. 3 is a schematic diagram showing the concept of deconvolution according to an embodiment of the present disclosure.

In FIG. 3, 100 is each peak that appears in the mass spectrum obtained by analyzing the entire sample material to be analyzed, and 200 is a peak that can appear in the theoretical mass spectrum according to the number of deuterium substitutions of a material having a specific chemical formula.

For the convenience of explanation, 200, which is a peak that can appear in the theoretical mass spectrum according to the number of deuterium substitutions of a material having a specific chemical formula is first described.

The molecular weight of C(i,n) is assumed to be M0.

If 210 in FIG. 3 is a theoretical peak represented by C(i,n) (mass number: M0), C(i,n) has the number of deuterium substitution fixed to i, and thus, each peak at 210 remains only the effect due to isotope (e.g., carbon isotope), which can be explained as follows.

M0: i.0) a peak due to $C(i,n)^{+\cdot}$ radical ion.

M0+1: i.1) a peak due to one isotope with mass number +1 (e.g., $^{13}C$) in $C(i,n)^{+}$

M0+2: i.2.1) a peak due to one isotope with mass number +2 (e.g., $^{14}C$) in $C(i,n)^{+\cdot}$; i.2.2) a peak due to two isotopes with mass number +1 (e.g., two $^{13}C$) in $C(i,n)^{+\cdot}$.

M0+3: 1.3.1) a peak due to one isotope with mass number +2 (e.g., $^{14}C$) and one isotope with mass number +1 (e.g., $^{13}C$) in $C(i,n)^{+\cdot}$; i.3.2) a peak due to three isotopes with mass number +1 (e.g., three $^{13}C$) in $C(i,n)^{+\cdot}$.

M0+4: i.4.1) a peak due to two isotopes with mass numbers +2 (e.g., two $^{14}C$) in $C(i,n)^{+\cdot}$, , i.4.2) a peak due to one isotope with mass numbers +2 (e.g., $^{14}C$) and two isotopes with mass numbers +1 (e.g., two $^{13}C$) in $C(i,n)^{+\cdot}$, i.4.3) a peak due to four isotopes with mass numbers +1 (e.g., four $^{13}C$) in $C(i,n)^{+\cdot}$.

If 220 in FIG. 3 is a theoretical peak appearing by C(i+1,n) (mass number: M0+1), C(i+1,n) has the number of

deuterium substitution fixed to i+1, and thus each peak of 220 remains only the effect due to carbon isotopes, which can be explained as follows.

M0: not appeared.

M0+1: i+1.0) a peak due to C(i+1,n)$^{+\cdot}$ radical ion.

M0+2: i+1.1) a peak due to one isotope with mass number +1 (e.g., $^{13}C$) in C(i+1,n)$^{+\cdot}$.

M0+3: i+1.2.1) a peak due to one isotope with mass number +2 (e.g., $^{14}C$) in C(i+1,n)$^{+\cdot}$; i+1.2.2) a peak due to two isotopes with mass number +1 (e.g., two $^{13}C$) in C(i+1,n)$^{+\cdot}$.

M0+4: i+1.3.1) a peak due to one isotope with mass number +2 (e.g., $^{14}C$) and one isotope with mass number +1 (e.g., $^{13}C$) in C(i+1,n)$^{+\cdot}$; i+1.3.2) a peak due to three isotopes with mass number +1 (e.g., three $^{13}C$) in C(i+1,n)$^{+\cdot}$.

If 230 in FIG. 3 is a theoretical peak appearing by C(i+2,n) (mass number: M0+2), C(i+2,n) has the number of deuterium substitution fixed to i+2, and thus each peak of 230 remains only the effect due to carbon isotopes, which can be explained as follows.

M0: not appeared.

M0+1: not appeared.

M0+2: i+2.0) a peak due to C(i+2,n)$^{+\cdot}$ radical ion.

M0+3: i+2.1) a peak due to one isotope with mass number +1 (e.g., $^{13}C$) in C(i+2,n)$^{+\cdot}$.

M0+4: i+2.2.1) a peak due to one isotope with mass number +2 (e.g., $^{14}C$) in C(i+2,n)$^{+\cdot}$; i+2.2.2) a peak due to two isotopes with mass number +1 (e.g., two $^{13}C$) in C(i+2,n)$^{+\cdot}$.

In consideration of the above, 100 in FIG. 3 is explained again as follows.

Assuming that the mass number eliminating the isotope effect (e.g., carbon isotope) in C(i,n) is M0, each peak at 100 in FIG. 3 appears by the sum of the following elements.

M0: i.0) a contribution by C(i,n)$^{+\cdot}$ radical ion.

M0+1: i.1) a contribution by one isotope with mass number +1 in C(i,n)+ · (e.g., $^{13}C$), and i+1.0) a contribution by C(i+1,n)$^{+\cdot}$ radical ion.

M0+2: i.2.1) a contribution by one isotope with mass number +2 (e.g., $^{14}C$) in C(i,n)$^{+\cdot}$, i.2.2) a contribution by two isotopes with mass number +1 (e.g., two $^{13}C$) in C(i,n)$^{+\cdot}$, i+1.1) a contribution by one isotope with mass number +1 (e.g., $^{13}C$) in C(i+1,n)$^{+\cdot}$, and i+2.0) a contribution by C(i+2,n)$^{+\cdot}$ radical ion.

M0+3: i.3.1) a contribution by one isotope with mass number +2 (e.g., $^{14}C$) and one isotope with mass number +1 (e.g., $^{13}C$) in C(i,n)$^{+\cdot}$, , i.3.2) a contribution by three isotopes with mass number +1 (e.g., three $^{13}C$) in C(i,n)$^{+\cdot}$, i+1.2.1) a contribution by one isotope with mass number +2 (e.g., $^{14}C$) in C(i+1,n)$^{+\cdot}$, , i+1.2.2) a contribution by two isotopes with mass number +1 (e.g., two $^{13}C$) in C(i+1,n)$^{+\cdot}$, and i+2.1) a contribution by one isotope with mass number +1 (e.g., $^{13}C$) in C(i+2,n)$^{+\cdot}$.

M0+4: i.4.1) a contribution by two isotopes with mass numbers +2 (e.g., two $^{14}C$) in C(i,n)$^{+\cdot}$, , i.4.2) a contribution by one isotope with mass numbers +2 (e.g., $^{14}C$) and two isotopes with mass numbers +1 (e.g., two $^{13}C$) in C(i,n)$^{+\cdot}$, , i.4.2) a contribution by four isotopes with mass numbers +1 (e.g., four $^{13}C$) in C(i,n)$^{+\cdot}$, i+1.3.1) a contribution by one isotope with mass numbers +2 (e.g., $^{14}C$) and one isotope with mass numbers +1 (e.g., $^{13}C$) in C(i+1,n)$^{+\cdot}$, , i+1.3.2) a contribution by three isotopes with mass numbers +1 (e.g., three $^{13}C$) in C(i+1,n)$^{+\cdot}$, i+2.2.1) a contribution by one isotope with mass numbers +2 (e.g., $^{14}C$) in C(i+2,n)$^{+\cdot}$, and i+2.2.2) a contribution by two isotopes with mass numbers +1 (e.g., two $^{13}C$) in C(i+2,n)$^{+\cdot}$.

However, as mentioned above, it is known that the natural abundance ratio of carbon ($^{12}C$) with a mass number of 12 is about 98.9%; the natural abundance ratio of carbon ($^{13}C$) with a mass number of 13 is about 1.1%; and the natural abundance ratio of carbon ($^{14}C$) with a mass number of 14 is about 0.0000000001%. Therefore, probabilistically, even if the contribution including $^{14}C$ or the contribution by two or more $^{13}C$ is ignored, the final analysis result may not differ significantly in arithmetic calculations, thereby simplifying the calculation.

Furthermore, according to the same principle, there may be no significant difference even if the peaks of M0+5 or more are ignored based on the chemical species C(i,n) with a mass number of M0.

That is, since each peak appearing in the mass spectrum obtained by analyzing the entire sample material to be analyzed is appeared by a linear combination of each peak in the mass spectrum that appears by each chemical species included in the sample material to be analyzed, this can be reversed and each peak appearing in the mass spectrum obtained by analyzing the entire sample material can be separated again into a linear combination of the mass spectrum peaks appearing by each of the chemical species contained in the sample material to be analyzed.

An example of such a method is least square approximation, etc. In the case of the least square approximation, the coefficient values used in the linear combination of each peak in the mass spectrum that appears by each chemical species, i.e., $a_i$, $a_{i+1}$, $a_{i+2}$, ... of 200 in FIG. 3 can be considered to be the ratio of each chemical species.

According to an embodiment of the present disclosure, the mass spectrum can be analyzed by the method as above to find out the ratio $a_0, a_1, a_2, a_3, ..., a_i, ..., a_{n-1}, a_n$ of each set element belonging to each chemical species included in the sample of the material be to analyzed, i.e., {C(0,n), C(1,n), C(2,n), C(3,n), ..., C(i,n), C(i+1,n), ..., C(n-1,n), C(n,n)}. When the set is rearranged by including the ratio of each chemical species, it can be expressed as follows. {C(0,n) $\times$ $a_0$,

$C(1,n) \times a1, C(2,n) \times a_2, C(3,n) \times a_3, ..., C(i,n) \times a_i, C(i+1,n) \times a_{i+1}, ..., C(n-1,n) \times a_{n-1}, C(n,n) \times a_n\}]$

According to an embodiment, the method for analyzing deuterium substitution rates of the sample material may further comprise a step 4 of calculating an average deuterium substitution rate of a sample material from the relative content values of each element chemical species belonging to the set.

According to an embodiment, the step 4 may calculate the average deuterium substitution rate of the sample material according to the following Mathematical Equation 1:

[Mathematical Equation 1]

$$SD(\%) = \frac{\sum_{i=0}^{n}(i \times P_i)}{n};$$

in Mathematical Equation 1,

SD(%) is the average deuterium substitution rate of the sample material being analyzed, n means the total number of hydrogen and deuterium bond sites in the compound C molecule, and $P_i$ is the relative amount(%) of a compound C(i,n) in which i of the total n hydrogen and deuterium bond sites are substituted by deuterium.

According to an embodiment, the $P_i$ may be calculated according to the following Mathematical Equation 2.

[Mathematical Equation 2]

$$P_i = \frac{a_i}{\sum_{i=0}^{n} a_i} \times 100$$

in Mathematical Equation 2, $P_i$ is the relative amount(%) of a compound C(i,n) in which i of the total n hydrogen and deuterium bond sites are substituted by deuterium, and $a_i$ is the weight of the C(i,n) molecule.

That is, the Mathematical Equation is merely an equation that arithmetically summarizes the relative ratio of each chemical species among the total amount of all chemical species included in the material sample to be analyzed, in the set $\{C(0,n) \times a_0, C(1,n) \times a1, C(2,n) \times a_2, C(3,n) \times a_3, ..., C(i,n) \times a_i, C(i+1,n) \times a_{i+1}, ..., C(n-1,n) \times a_{n-1}, C(n,n) \times a_n\}$ found by the analysis method described above, and the present disclosure is not necessarily limited thereto.

Meanwhile, provided herein is a method for predicting deuterium substitution rates of a reaction product, comprising: a step of measuring an average deuterium substitution rate of a first reactant by the above-mentioned method, a step of measuring an average deuterium substitution rate of a second reactant by the above-mentioned method, and a step of predicting a deuterium substitution rate of a reaction product obtained by the reaction between the first reactant and the second reactant using the average deuterium substitution rate of the first reactant and the average deuterium substitution rate of the second reactant.

That is, according to the above method, in a chemical reaction in which a specific reactant substituted with deuterium react to form a reaction product, it is possible to predict the relative amounts by the number of substitutions of each chemical species existing in the reaction product and the average deuterium substitution rate for all chemical species by utilizing the relative amount and average substitution rate by the number of deuterium substitutions obtained through deuterium substitution rate analysis, even when a separate analysis of the reaction product is not performed.

Hereinafter, a reaction in which two molecules react to form one molecule will be described as an example.

The reaction in which two molecules substituted with deuterium at specific positions react to form one molecule can be classified into the following three cases.

> i) A first case in which a functional group at a specific position of a compound 1-1 is replaced by a compound 1-2, when one deuterium-substituted compound (hereinafter, compound 1-1) and one deuterium-unsubstituted compound (hereinafter, compound 1-2) react to form a product (hereinafter, compound 1);
> ii) A second case in which the deuterium substitution position of a compound 2-1 is maintained as it is, when one deuterium-substituted compound (hereinafter, compound 2-1) and one deuterium-unsubstituted compound (hereinafter, compound 2-2) react to form a product (hereinafter, compound 2);
> iii) A third case in which one deuterium-substituted compound (hereinafter, compound 3-1) reacts with another deuterium-substituted compound (hereinafter, compound 3-2) to form a product (hereinafter, compound 3).

The first case will be described first.

FIG. 4 is a conceptual diagram for explaining changes in deuterium substitution characteristics in a reaction

according to an embodiment.

In FIG. 4, the compound 1-1 is a deuterium compound which is substituted with deuterium, the compound 1-2 is a molecule which is not substituted with deuterium, and the compound 1-2 is substituted at a specific substitution position X of the compound 1-1 to form a compound 1.

In this case, the probability that the X position of the compound 1-1 is 1-hydrogen is a. The value of a can arithmetically use the "average substitution rate" value of the deuterium-substituted reaction product obtained through the above-mentioned mass analysis, or can use the substitution rate value obtained through other methods, such as NMR analysis.

In this case, the relative amount P() for each deuterium substitution number in the compound 1, which is a product, can be expressed by the following Mathematical Equation 3-1.

[Mathematical Equation 3-1]

$$P_i(\text{compound 1}) = \{P_{i+1}(\text{compound 1-1}) \times (1\text{-a})\} + \{P_i(\text{compound 1-1}) \times a\}$$

In the above Equation, P() is the relative amount of molecule in which deuterium is substituted at position i, and a is the probability that original hydrogen exists at the substitution position X of the compound 1-1.

In this case, the average deuterium substitution rate (SD1) of the product can be expressed by the following Mathematical Equation 3-2.

[Mathematical Equation 3-2]

$$SD1(\%) = \frac{\sum_{i=0}^{n}(i \times P_i(\text{Compound 1}))}{n}$$

Next, the second case will be described.

FIG. 5 is a conceptual diagram for explaining changes in deuterium substitution characteristics in a reaction according to an embodiment.

In FIG. 5, the compound 2-1 is a compound which is substituted with deuterium the 2-2 compound is a molecule which is not substituted with deuterium, the portion of the substitution position of the compound 2-1 where the substituent X was originally connected is substituted by the 2-2 compound to form the compound 2.

In this case, the relative amount P() of deuterium substitutions in the compound 2, which is the product, is identical to the relative amount P() for each number of deuterium substitutions in the compound 2-1, which is the reactant, and thus it can be expressed by the following Mathematical Equation 4-1.

[Mathematical Equation 4-1]

$$P_i(\text{Compound 2}) = P_i(\text{Compound 2-1})$$

$$SD2(\%) = \frac{\sum_{i=0}^{n}(i \times P_i(\text{Compound 2}))}{n}$$

In this case, the average deuterium substitution rate (SD2) of the product can be expressed by the following Mathematical Equation 4-2.

[Mathematical Equation 4-2]

Next, the third case will be explained.

FIG. 6 is a conceptual diagram for explaining changes in deuterium substitution characteristics in a reaction according to an embodiment.

In FIG. 6, the compound 3-1 and the compound 3-2 are deuterium compounds which is substituted with deuterium, and among the substitution positions of the compound 3-1, the portion where the original substituent X

was connected is substituted with a compound 3-2 to form a compound 3.

In this case, in the compound 3, which is a product, the relative amount P() according to the number of deuterium substitutions can be expressed by the following Mathematical Equation 5-1.

[Mathematical Equation 5-1]

$P_i$ (Compound 3) = sigma (m = 0 to i) {Pm(Compound 3-1) × Pi-m(Compound 3-2) }

In this case, the average deuterium substitution rate (SD3) of the product can be expressed by the following mathematical Equation 5-2.

[Mathematical Equation 5-2]

$$SD3(\%) = \frac{\sum_{i=0}^{n}(i \times P_i(\text{Compound 3}))}{n}$$

When a deuterium compound reacts to produce another deuterium compound by using the method as above, the relative amount P() of each number of deuterium substitution in the product and the average deuterium substitution rate of the product can be predicted through the deuterium substitution analysis result of the reactant even when a separate deuterium substitution analysis on the product is not performed.

[Advantageous Effects]

According to an embodiment of the present disclosure, the relative amount of each number of deuterium substitution and the average deuterium substitution rate in the sample to be analyzed can be accurately found out through MS analysis, and the relative amount of each number of deuterium substitution P() and the average deuterium substitution rate of the product can be predicted through the deuterium substitution analysis results of the reactant, even when a separate deuterium substitution analysis on the product is not performed.

[BRIEF DESCRIPTION OF THE DRAWINGS]

FIGS. 1 and 2 are examples of typical mass spectra obtained by MALDI-TOF MS analysis.
FIG. 3 is a schematic diagram showing the concept of deconvolution according to an embodiment of the present disclosure.
FIGS. 4 to 6 are conceptual diagrams for explaining changes in deuterium substitution characteristics in a reaction according to an embodiment.
FIG. 7 is a MALDI-TOF mass spectrum for a compound according to an embodiment.
FIG. 8 is a MALDI-TOF mass spectrum for a compound according to an embodiment.
FIG. 9 is an NMR analysis result for a compound according to an embodiment.
FIG. 10 is a MALDI-TOF mass spectrum for a compound according to an embodiment.

[DETAILED DESCRIPTION OF THE EMBODIMENTS]

Hereinafter, the action and effect of the invention will be described in more detail by way of specific examples. However, these examples are presented for illustrative purposes only, and the scope of the invention is not defined by them.

<EXAMPLE>

In the reaction of the compound shown below, the deuterium substitution rate of the precursor and the

intermediate (reactant) was analyzed, while the deuterium substitution rate of the product produced by the reaction between the first intermediate and the second intermediatewas predicted.

First precursor:

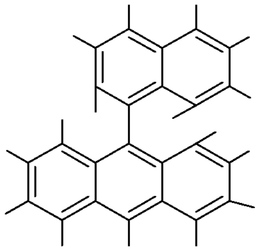

Formula: 16
Exact 320.23

First intermediate:

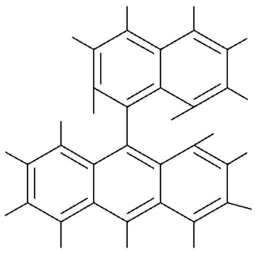

Formula: 16
Exact 320.23

Second precursor:

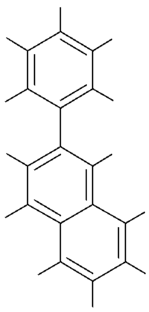

Formula: 16
Exact 320.23

Second intermediate:

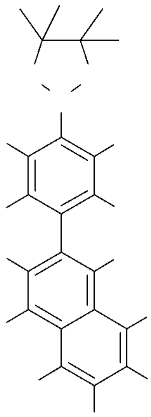

Formula: 16 BO
Exact 320.23

Reaction schematic diagram:

First precursor->First intermediate; Second precursor->Second intermediate;

First intermediate + Second intermediate->Product:

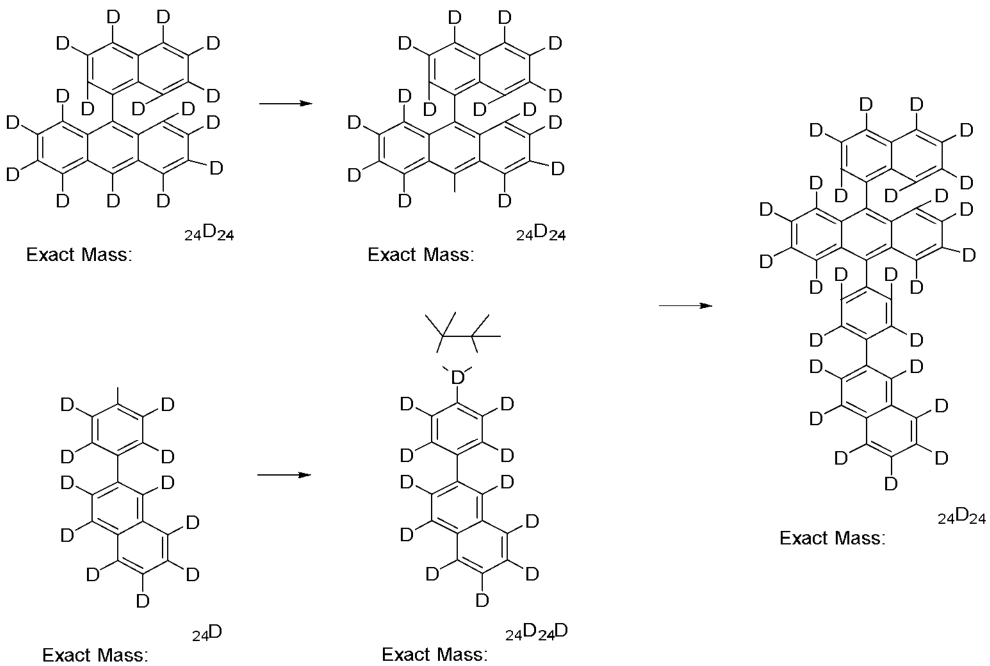


First, a pre-experimentation was conducted to select an appropriate matrix for the analysis.

## Preparation of analysis sample (Preparation Example)

1 mg of $Al_2O_3$ powder was added to 300 $\mu$L of tetrahydrofuran (THF) to prepare an $Al_2O_3$ matrix solution.
Separately, 1 mg of the material to be analyzed (H-form of the second intermediate) was dissolved in 1 mL of THF to prepare a material solution to be analyzed.
The $Al_2O_3$ matrix solution was shaken, 10 $\mu$L of the solution was taken in state in which $Al_2O_3$ was dispersed, and this was mixed with 10 $\mu$L of the material solution to be analyzed to prepare a mixed solution.
1 $\mu$L of the mixed solution was taken and placed on a MALDI sample plate and naturally dried to prepare a

specimen.

## Preparation of analysis sample (Reference Preparation Example)

1 mg of dihydroxybenzoic acid was added to 100 μL of tetrahydrofuran(THF) to prepare an organic matrix solution.

Separately, 1 mg of the material to be analyzed (H-form of the second intermediate) was dissolved in 1 mL of THF to prepare a material solution to be analyzed.

10 μL of the organic matrix solution was taken and mixed with 10 μL of the analyte solution to prepare a mixed solution.

1 μL of the mixed solution was taken and placed on a MALDI sample plate and naturally dried to prepare a specimen.

## Acquisition and analysis of mass spectrum

### MALDI-TOF MS experimental conditions:

Sample rate: 2.5 GS/s or higher; Laser repetition rate: 100 or 200 Hz; Laser condition: Shot at raster spot (50), Limit diameter (500 μm); Mass spectrometer: positive mode

FIG. 7 is a MALDI-TOF mass spectrum for the second intermediate(H-form).

Referring to FIG. 7, it can be confirmed that in the case of Reference Preparation Example using an organic matrix, the peak intensity of 331 is increased compared to Preparation Example using an inorganic oxide matrix.

This is considered to be an effect due to the increase in mass number that appears when another hydrogen is further bonded during analysis. In this way, chemical species in which hydrogen is further bonded to the molecule can be generated according to the type of matrix, and therefore it is difficult to eliminate the effect of a change in mass number. In other words, when analyzing a compound substituted with deuterium, the effect of the increase in mass number due to hydrogenation is reflected in addition to the effects of the increase in mass number by 1 due to deuterium substitution and the increase in mass number due to isotopes of elements other than heavy hydrogen that constitute the compound, which may reduce the accuracy of the analysis.

Therefore, in the following experiments of this example, an inorganic oxide series matrix was used to conduct the experiment.

## Preparation of analysis sample

1 mg of $Al_2O_3$ powder was added to 300 μL of tetrahydrofuran (THF) to prepare an $Al_2O_3$ matrix solution.

Separately, 1 mg of the material to be analyzed was dissolved in 1 mL of THF to prepare a material solution to be analyzed.

The $Al_2O_3$ matrix solution was shaken, 10 μL of the solution was taken in state in which $Al_2O_3$ was dispersed, and this was mixed with 10 μL of the material solution to be analyzed to prepare a mixed solution.

1 μL of the mixed solution was taken and placed on a MALDI sample plate and naturally dried to prepare a specimen.

## Acquisition and analysis of mass spectrum

### MALDI-TOF MS experimental conditions:

Sample rate: 2.5 GS/s or higher; Laser repetition rate: 100 or 200 Hz; Laser condition: Shot at raster spot (50), Limit diameter (500 μm); Mass spectrometer: positive mode

## Analysis of average deuterium substitution rate of the first precursor

### (Mass spectrometry results)

FIG. 8 is a MALDI-TOF mass spectrum for the first precursor.

In the first precursor compound, the number of deuterium substitution sites is 16 in total, and the number of carbons is 24, so that each compound contained in the first precursor compound can be expressed by C(i,16), where i= 0 to 16.

In this analysis result, since the effect due to hydrogen ($^1H$) mentioned above can be eliminated, only the effect

due to the deuterium substitution number can be left if the effect due to isotopes can be eliminated here. Since the first precursor compound contains only carbon in addition to hydrogen or deuterium, the effect due to the deuterium substitution number can be confirmed if the effect due to carbon isotopes can be eliminated in the analysis result.

Using the natural abundance ratio of carbon ($^{12}C$) of about 98.9%; the natural abundance ratio of carbon ($^{13}C$) of about 1.1%; and the natural abundance ration of mass carbon ($^{14}C$) of about 0.0000000001%, the effect due to carbon isotopes is eliminated in the mass spectrum of FIG. 8, and this is again separated into a linear combination of each peak of the mass spectrum appearing for each chemical species, and each coefficient value is calculated, rounded to the first decimal place, and summarized in Table 1 below.

{C(0,16) $\times$ $a_0$, C(1,16)$\times$ a1, C(2,16)$\times$ $a_2$, C(3,16)$\times$ $a_3$, ..., C(i,16)$\times$ $a_i$, C(i+1,16)$\times$ $a_{i+1}$, ..., C(15,16)$\times$ $a_{15}$, C(16,16)$\times$ $a_{16}$}

[Table 1]

| Number of deuterium substitutions (i) | 12 | 13 | 14 | 15 | 16 |
|---|---|---|---|---|---|
| Relative ratio (Pi) | 2 | 11 | 29 | 40 | 18 |

The average deuterium substitution rate was calculated using Mathematical Equation 1 and as a result, calculated to be about 91.2%.

[Mathematical Equation 1]

$$SD(\%) = \frac{\sum_{i=0}^{n}(i \times P_i)}{n};$$

wherein, n=16.

FIG. 9 shows the NMR analysis result of the first precursor.

The result of the NMR analysis of FIG. 9 shows that the probability a of the ring position A-1 of the first precursor being 1-hydrogen is about 6.8%.

<u>**Prediction of the average deuterium substitution rate of the first intermediate**</u>

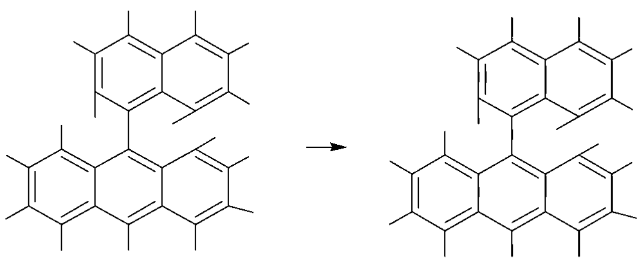

Exact 320.23 Exact 320.23

In the reaction, the reaction site of the first precursor is a site where deuterium substitution is possible, and this site is substituted with Br in the first intermediate. Since this corresponds to the first case mentioned above, the average substitution rate of the first intermediate can be calculated through Mathematical Equations 3-1 and 3-2.

In the following Mathematical Equation 3-1, the probability a that the ring position A-1 of the first precursor is 1-hydrogen may use the "average substitution rate" value of the deuterium substitution reaction product obtained through the mass analysis, or can use the substitution rate value obtained through the NMR analysis, etc.

$P_i$ (First intermediate) = { $P_i$ + 1 (First precursor) $\times$ (1-a) } + { $P_i$ (First precursor) $\times$ a } [Mathematical Equation 3-1]

And, the average deuterium substitution rate (SD1) of the first intermediatecan be obtained by the following Mathematical Formula 3-2.

[Mathematical Equation 3-2]

$$SD1(\%) = \frac{\sum_{i=0}^{n}(i \times P_i(\text{Compound 1}))}{n}$$

wherein, n=15.

In Table 2 below, the values measured by actual experiments for the first intermediate and the values predicted by the above method are summarized together.

[Table 2]

| Number of deuterium substitutions (i) | 11 | 12 | 13 | 14 | 15 | Average substitution rate |
|---|---|---|---|---|---|---|
| Relative ratio (Experimental measurement value) | - | 8 | 28 | 40 | 24 | 91.9 % |
| Relative ratio (Predicted value*) | 2 | 10 | 27 | 39 | 22 | 91.2 % |
| Relative ratio (Predicted value**) | 2 | 10 | 28 | 39 | 21 | 91.1 % |
| *When calculating the predicted value, the average substitution rate value obtained through mass spectrometry is used as the value a in the Mathematical Equation 3-1. **When calculating the predicted value, the deuterium substitution rate value at the functional group substitution site (X) obtained through NMR analysis is used as the value a in the Mathematical Equation 3-1, | | | | | | |

According to the Table 2, it can be confirmed that the tendency of the ratio according to each number of substitution is the same between the values measured by the actual experiment and the predicted values, and that there is almost no error in the value of the obtained average substitution rate.

**Analysis of the average deuterium substitution rate of the second precursor**

FIG. 10 is a MALDI-TOF mass spectrum for the second precursor.

In the secnod precursor compound, the number of deuterium substitution sites is 11 in total and the number of carbons is 16, so that each compound contained in the precursor 2 compound can be expressed by C(i,11), where i=0 to 11.

In this analysis result, since the effect due to hydrogen ($^1H$) mentioned above can be eliminated, only the effect due to the number of deuterium substitutions can be remained if the effect due to isotopes can be eliminated here. Since the second precursor compound contains carbon and chlorine in addition to hydrogen or deuterium, if the effect due to the isotopes of carbon and chlorine can be eliminated in the analysis result, the effect due to the number of deuterium substitutions can be confirmed.

Using the natural abundance ratio of $^{12}C$ of about 98.9%; the natural abundance ratio of $^{13}C$ of about 1.1%; the natural abundance ratio of $^{14}C$ of about 0.0000000001%; the natural abundance ratio of $^{35}Cl$ of about 75.8%; and the natural abundance ratio of $^{37}Cl$ of about 24.2%, the effects due to carbon and chlorine isotopes were eliminated from the mass spectrum in FIG. 10, each peak of the mass spectrum was separated into a linear combination of each chemical species, and each coefficient value was calculated, rounded to the first decimal place, and summarized in Table 3.

{C(0,11) $\times$ $a_0$, C(1,11)$\times$ a1, C(2,11)$\times$ $a_2$, C(3,11)$\times$ $a_3$, ..., C(i,11)$\times$ $a_i$, C(i+1,11)$\times$ $a_{i+1}$, ..., C(10,11)$\times$ $a_{10}$, C(11,11)$\times$ $a_{11}$}

[Table 3]

| Number of deuterium substitutions (i) | 6 | 7 | 8 | 9 | 10 | 11 |
|---|---|---|---|---|---|---|
| Relative ratio (Pi) | 1 | 7 | 22 | 36 | 30 | 4 |

And, the average deuterium substitution rate was calculated using Mathematical Equation 1 and as a result, calculated to be about 81.7%.

[Mathematical Equation 1]

$$SD(\%) = \frac{\sum_{i=0}^{n}(i \times P_i)}{n};$$

wherein, n=11.

**Prediction of the average deuterium substitution rate of the second intermediate**

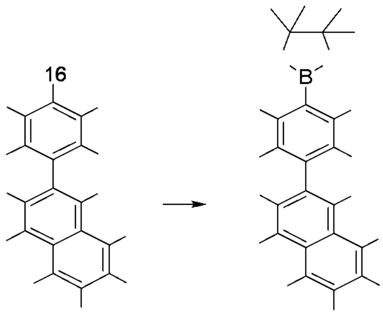

In the reaction, the reaction site of the second precursor is the site where Cl was located, and since this site is substituted by the tetramethyl-1,3,2-dioxaborolan compound in the second intermediate, this corresponds to the second case mentioned above.

Therefore, if the deuterium substitution form and ratio of the second intermediate are predicted using the results of Table 3 above, they can be expressed as in Table 4 below.

In Table 4 below, the values according to the actual experimental results and the predicted values are summarized together.

[Table 4]

| Number of deuterium substitutions (i) | 6 | 7 | 8 | 9 | 10 | 11 | Substitution rate |
|---|---|---|---|---|---|---|---|
| Relative ratio (Experiment ) | 1 | 5 | 22 | 37 | 31 | 4 | 82.0% |
| Relative ratio (Prediction) | 1 | 7 | 22 | 36 | 30 | 4 | 81.7% |

According to Table 4, it can be confirmed that the tendency of the ratio by each number of substitution appears equally between the actual experimental value and the predicted value, and that there is almost no error in the average substitution rate value.

**Prediction of the average deuterium substitution rate of the product**

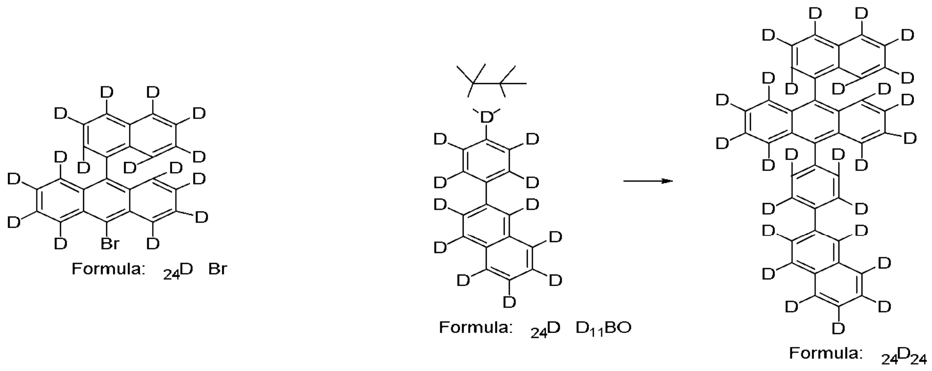


In the reaction, a product is formed by the reaction between the intermediate 1 and the second intermediate.

The reaction site of the first intermediate is the site where Br was located, and the reaction site of the second intermediate is the site which was substituted with the tetramethyl-1,3,2-dioxaborolan compound, and therefore, this corresponds to the third case mentioned above.

Therefore, if the deuterium substitution form and ratio of the product are predicted using the results of Tables 2 and 4, they can be expressed as in Table 5 below.

In Table 5 below, the values according to the actual experimental results and the predicted values are summarized together.

[Table 5]

| Number of deuterium substitutions (i) | Relative ratio (Experiment ) | Relative ratio (Prediction) |
|---|---|---|
| 19 | 1 | 1 |
| 20 | 4 | 5 |
| 21 | 12 | 13 |
| 22 | 25 | 23 |
| 23 | 28 | 28 |
| 24 | 23 | 21 |
| 25 | 7 | 8 |
| 26 | - | 1 |
| Average substitution rate | 87.3% | 87.2% |

According to Table5, it can be confirmed that the tendency of the ratio according to each number of substitution is the same between the actual experimental value and the predicted value, and also that there is almost no error in the average substitution rate values.

## Claims

**1.** A method for analyzing deuterium substitution rates of a sample material, comprising:

a step 1 of obtaining a mass spectrum for a sample of an analysis object material including one or more compounds selected from the set of chemical species represented by {C(0,n), C(1,n), C(2,n), C(3,n), ..., C(i,n), C(i+1,n), ..., C(n-1,n), C(n,n)};
a step 2 of expressing the mass spectrum of the sample material as a linear combination of the mass spectra of each of element compound belonging to the set; and
a step 3 of calculating the relative content value of each element compound belonging to the set from the coefficients of the linear combination:

in the compound C(i,n) as each element constituting the set,
C means that the compounds as each element constituting the set are identical compounds except for

whether they are substituted by hydrogen or deuterium,
n means the total number of hydrogen and deuterium bond sites in the compound C molecule,
i means the number of deuterium substitutions in the element compound included in the set, and
C(i,n) means a compound in which i of the total n hydrogen and deuterium bond sites are substituted by deuterium.

2. The method for analyzing deuterium substitution rates of a sample material according to claim 1, wherein the step 1 is a step of obtaining a mass spectrum by MALDI-TOF MS measurement using an inorganic oxide matrix.

3. The method for analyzing deuterium substitution rates of a sample material according to claim 2, wherein the inorganic oxide matrix is an inorganic oxide matrix including at least one element selected from the group consisting of silicon, zirconium, aluminum, and cerium.

4. The method for analyzing deuterium substitution rates of a sample material according to claim 1, wherein the step 2 further comprises a step of eliminating the effects due to isotope using the natural abundance ratio of isotopes.

5. The method for analyzing deuterium substitution rates of a sample material according to claim 1, wherein the step 2 expresses the mass spectrum of the sample material as a linear combination of the mass spectra of each of the element compounds belonging to the set using least square approximation.

6. The method for analyzing deuterium substitution rates of a sample material according to claim 1, further comprising a step 4 of calculating an average deuterium substitution rate of a sample material from the relative content values of each of the element compounds belonging to the set.

7. The method for analyzing deuterium substitution rates of a sample material according to claim 6, wherein the step 4 calculates the average deuterium substitution rate of the sample material according to the following Mathematical Equation 1:

[Mathematical Equation 1]

$$SD(\%) = \frac{\sum_{i=0}^{n}(i \times P_i)}{n};$$

in Mathematical Equation 1,

SD(%) is the average deuterium substitution rate of the sample material to be analyzed,
n means the total number of hydrogen and deuterium bond sites in the compound C molecule, and
$P_i$ is the relative amount(%) of a compound C(i,n) in which i of the total n hydrogen and deuterium bond sites are substituted by deuterium.

8. The method for analyzing deuterium substitution rates of a sample material according to claim 7, wherein the $P_i$ is calculated according to the following Mathematical Equation 2:

[Mathematical Equation 2]

$$P_i = \frac{a_i}{\sum_{i=0}^{n} a_i} \times 100$$

in Mathematical Equation 2,

$P_i$ is the relative amount(%) of a compound C(i,n) in which i of the total n hydrogen and deuterium bond sites are substituted by deuterium, and
$a_i$ is the weight of the C(i,n) molecule.

**9.** A method for predicting deuterium substitution rates of a reaction product, comprising:

a step of measuring an average deuterium substitution rate of a first reactant by the method according to claim 1,
a step of measuring an average deuterium substitution rate of a second reactant by the method according to claim 1, and
a step of predicting a deuterium substitution rate of a reaction product obtained by the reaction between the first reactant and the second reactant using the average deuterium substitution rate of the first reactant and the average deuterium substitution rate of the second reactant.

*FIG. 1*

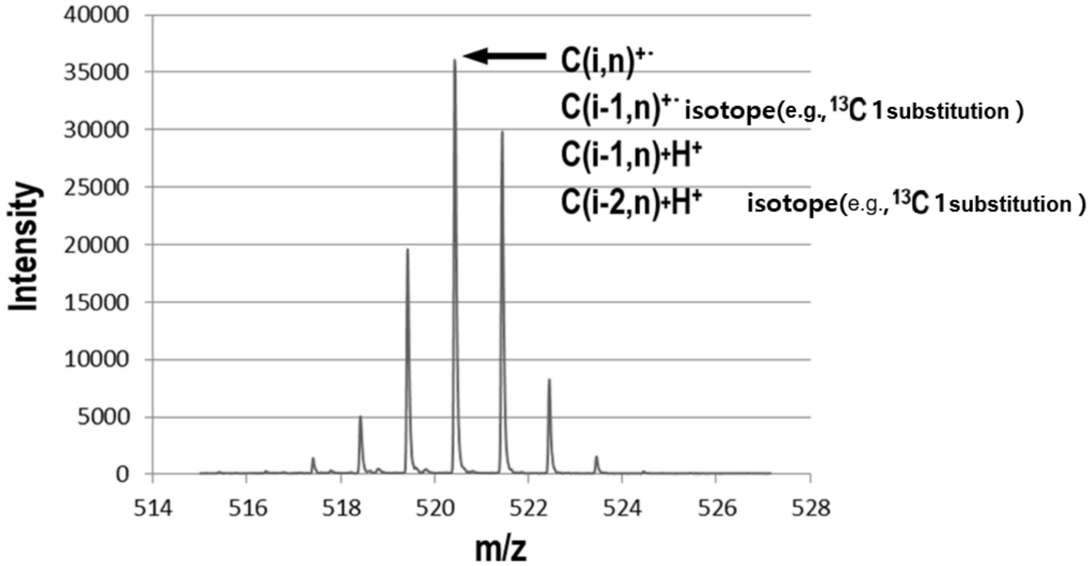
Intensity
m/z
40000
35000
30000
25000
20000
15000
10000
5000
0
514
516
518
520
522
524
526
528
C(i,n)+·
C(i-1,n)+· isotope(e.g., 13C 1 substitution )
C(i-1,n)+H+
C(i-2,n)+H+ isotope(e.g., 13C 1 substitution )

*FIG. 2*

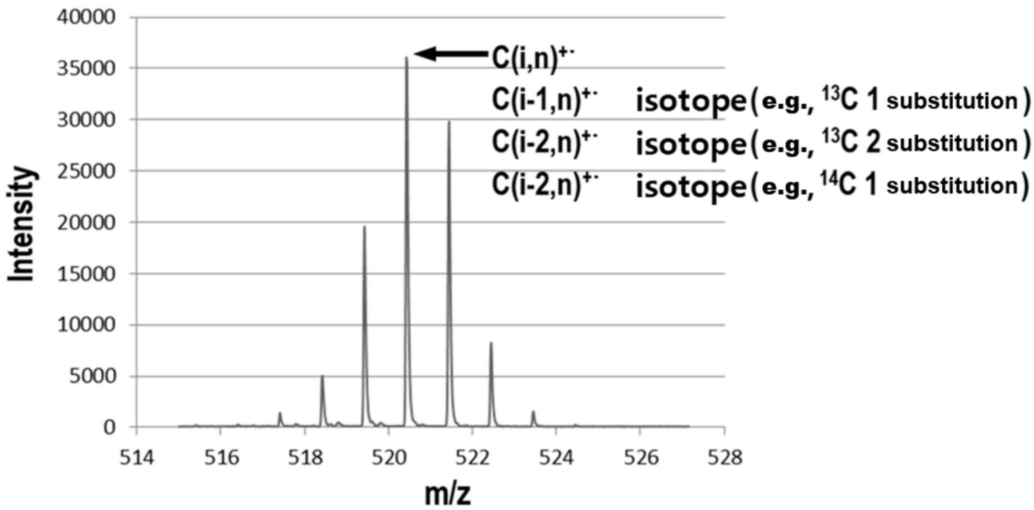
Intensity
m/z
40000
35000
30000
25000
20000
15000
10000
5000
0
514
516
518
520
522
524
526
528
C(i,n)+·
C(i-1,n)+· isotope(e.g., 13C 1 substitution)
C(i-2,n)+· isotope(e.g., 13C 2 substitution)
C(i-2,n)+· isotope(e.g., 14C 1 substitution)

*FIG. 3*

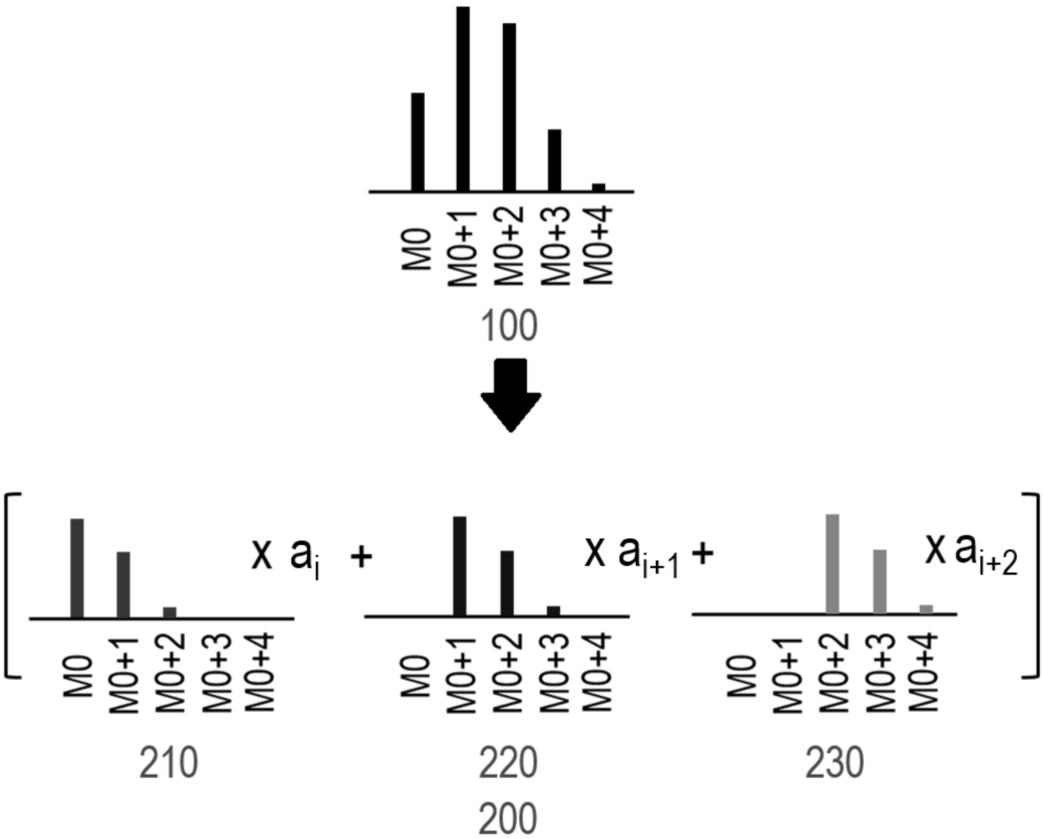
M0
M0+1
M0+2
M0+3
M0+4
100
x $a_i$
+
x $a_{i+1}$
+
x $a_{i+2}$
210
220
230
200

*FIG. 4*

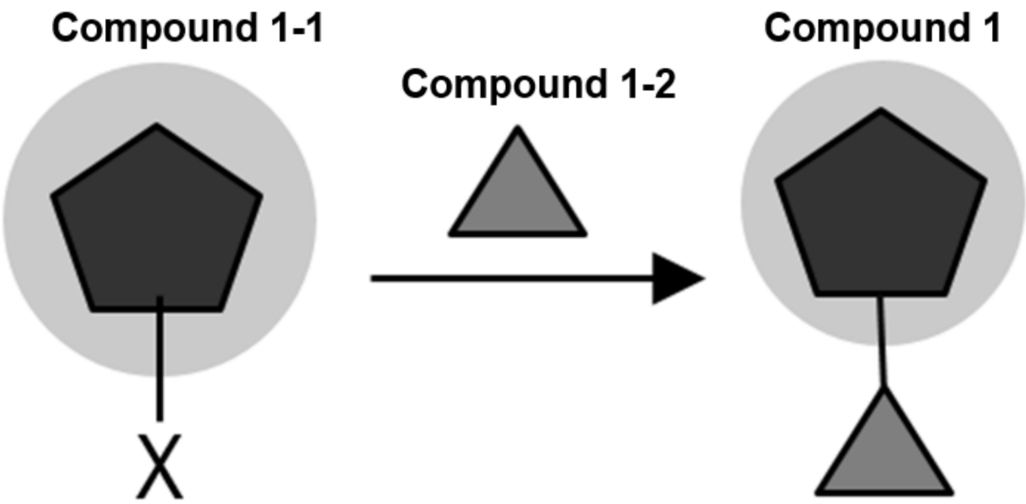
Compound 1-1
Compound 1-2
Compound 1
X

*FIG. 5*

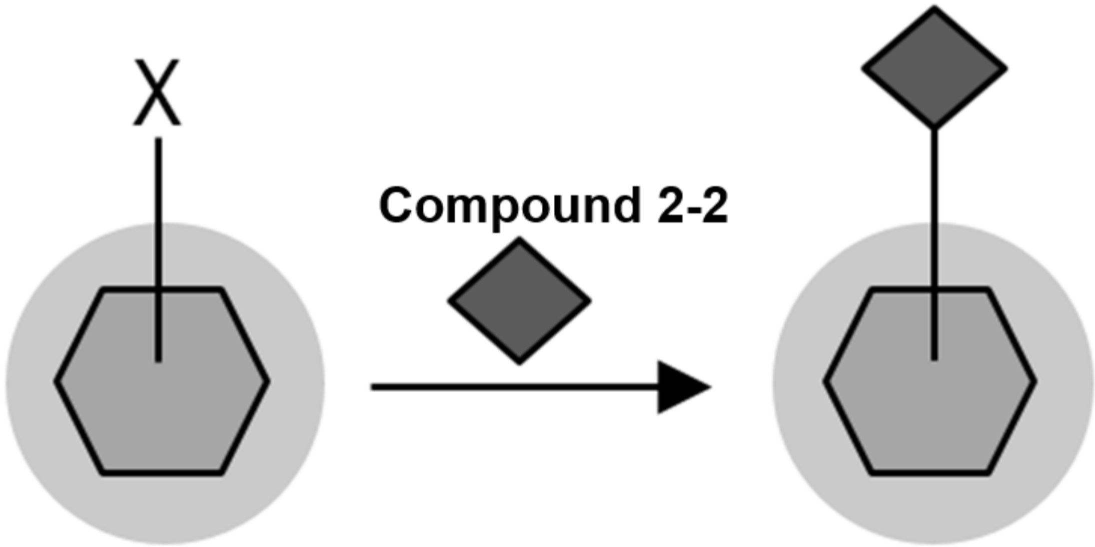


Compound 2-1 Compound 2

*FIG. 6*

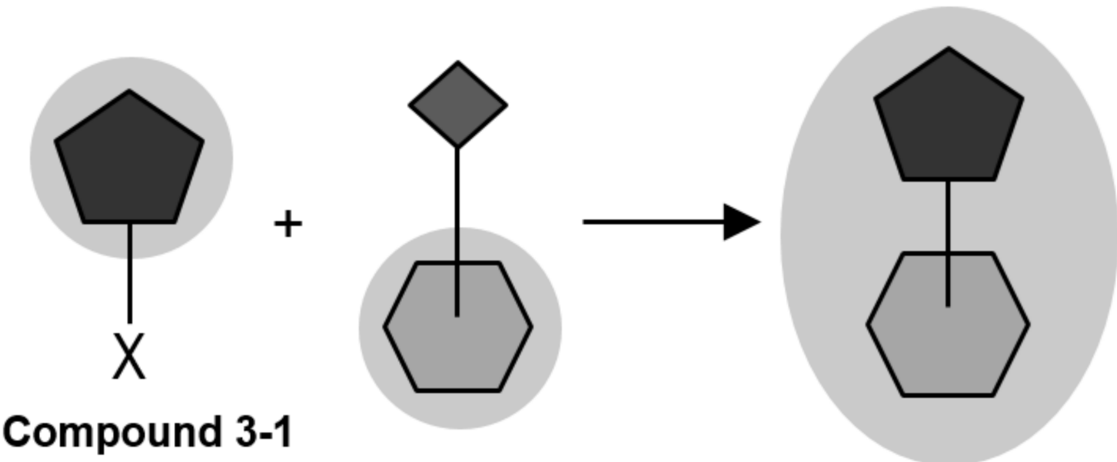


Compound 3-2 Compound 3

*FIG. 7*

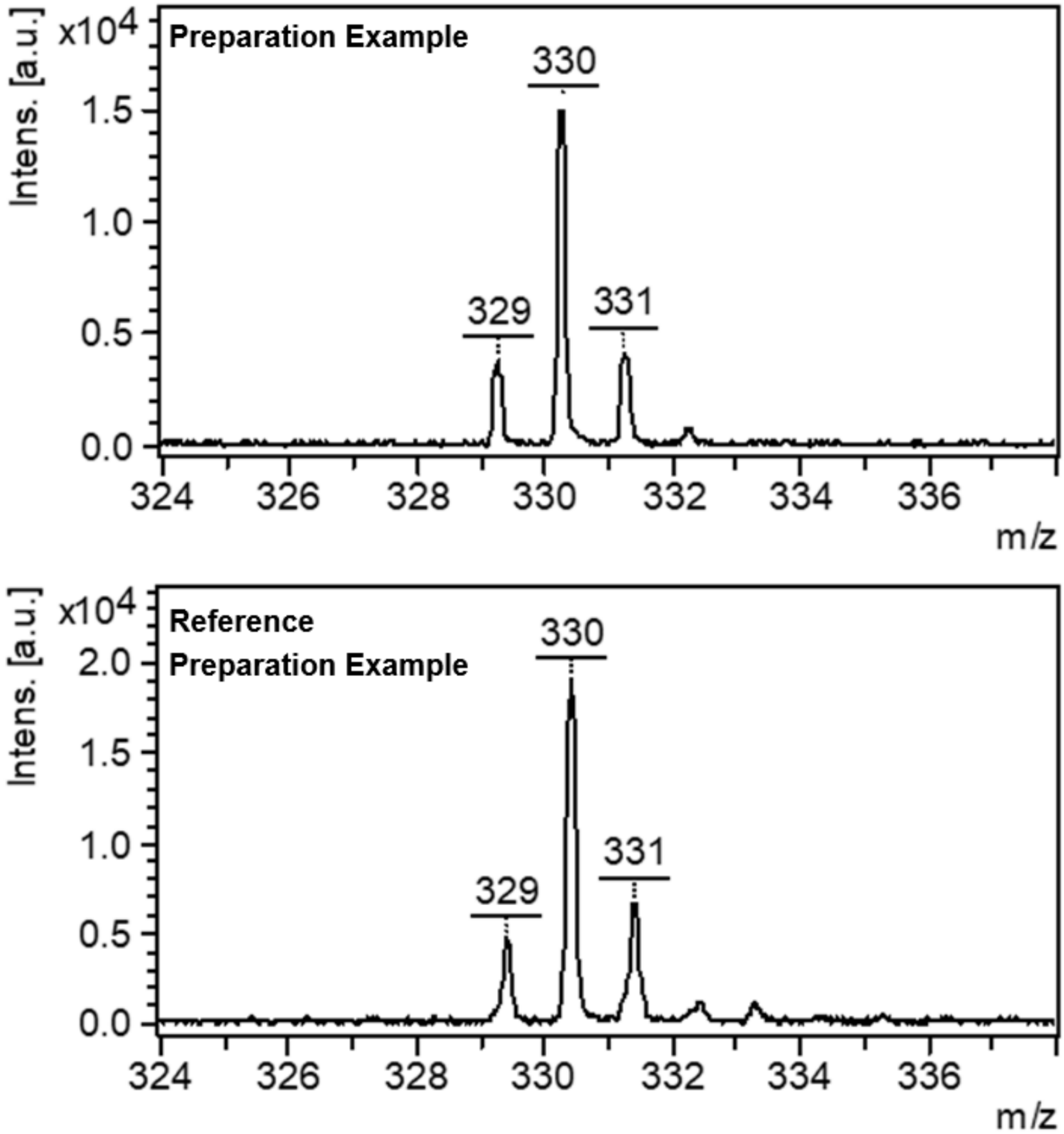
Intens. [a.u.]
x10^4
Preparation Example
330
329
331
1.5
1.0
0.5
0.0
324
326
328
330
332
334
336
m/z
Intens. [a.u.]
x10^4
Reference
Preparation Example
330
329
331
2.0
1.5
1.0
0.5
0.0
324
326
328
330
332
334
336
m/z

*FIG. 8*

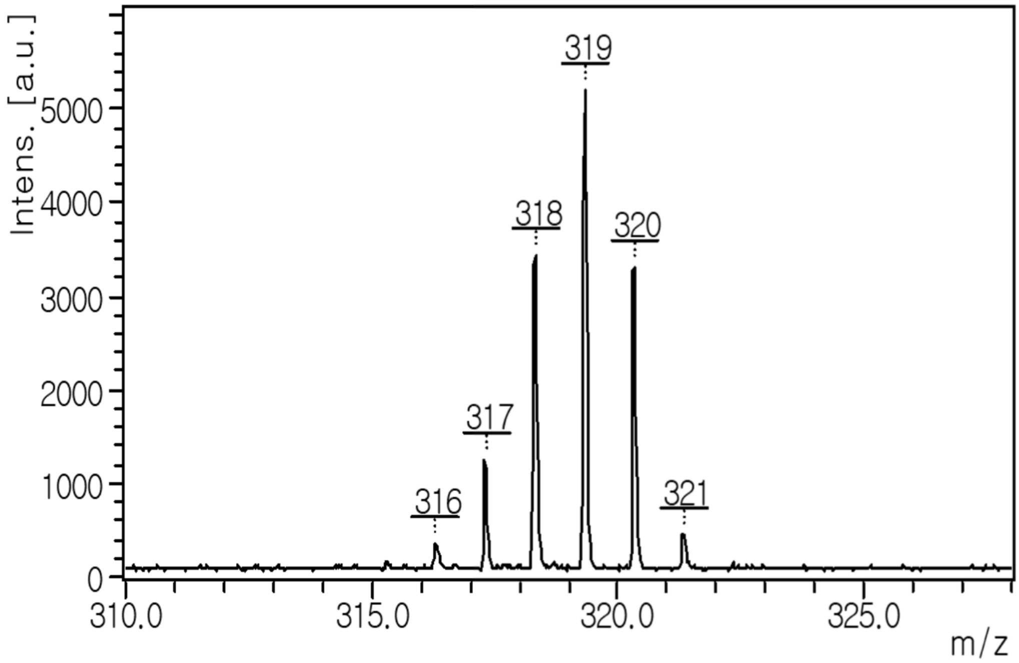


*FIG. 9*

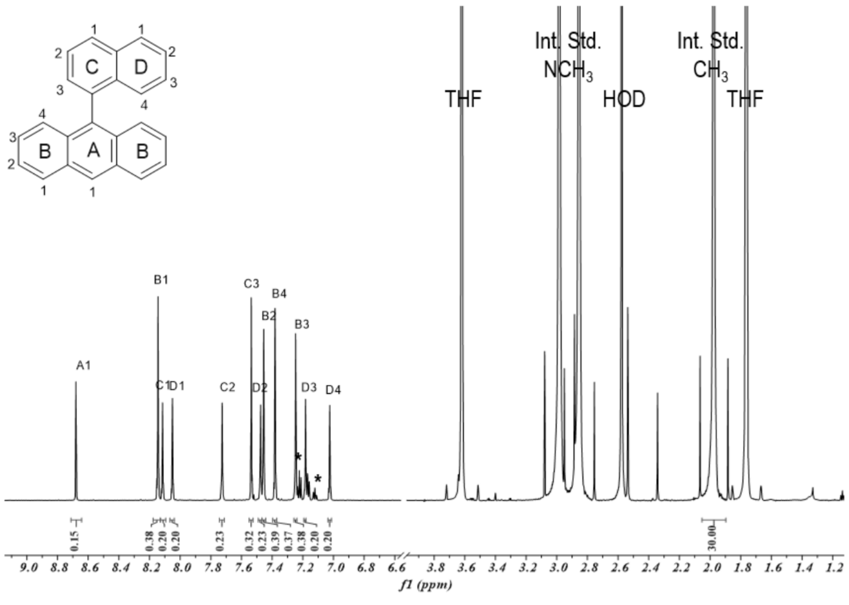


| Position | A1 |
|---|---|
| Number of H | 1 |
| D-Substitution Rate(%) | 93.2 |

*FIG. 10*

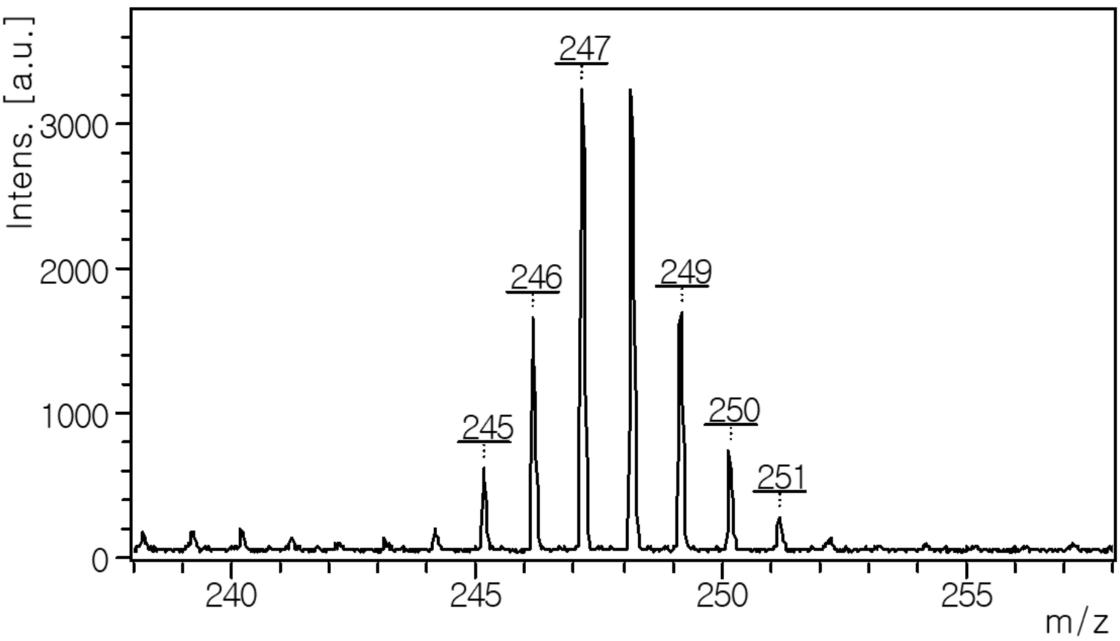
Intens. [a.u.]
3000
2000
1000
0
247
246
249
245
250
251
240
245
250
255
m/z

## INTERNATIONAL SEARCH REPORT

International application No. **PCT/KR2024/008466**

**A. CLASSIFICATION OF SUBJECT MATTER**

**G01N 27/623**(2021.01)i; **H01J 49/04**(2006.01)i; **H01J 49/16**(2006.01)i; **H01J 49/40**(2006.01)i

According to International Patent Classification (IPC) or to both national classification and IPC

**B. FIELDS SEARCHED**

Minimum documentation searched (classification system followed by classification symbols)

G01N 27/623(2021.01); G01N 24/08(2006.01); G01N 27/62(2006.01); G01N 27/64(2006.01); G01N 30/72(2006.01); G01N 30/86(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 중수소(deuterium), 치환(substitution), MALDI-TOF MS, 선형 결합(linear combination), 함량(content)

**C. DOCUMENTS CONSIDERED TO BE RELEVANT**

| Category* | Citation of document, with indication, where appropriate, of the relevant passages | Relevant to claim No. |
|---|---|---|
| X<br>Y<br>A | KR 10-2022-0017749 A (LG CHEM, LTD.) 14 February 2022 (2022-02-14)<br>See paragraphs [0073] and [0106]-[0125], table 2 and claims 1 and 2. | 1,5-8<br>2-4<br>9 |
| Y | JP 2009-505055 A (UNIVERSITY OF SUNDERLAND) 05 February 2009 (2009-02-05)<br>See abstract, paragraphs [0026] and [0039] and claims 2-5. | 2,3 |
| Y | SALISBURY et al. QUDeX-MS: hydrogen/deuterium exchange calculation for mass spectra with resolved isotopic fine structure. BMC bioinformatics. 11 December 2014, vol. 15, pp. 1-13.<br>See pages 3 and 4 and figure 2. | 4 |
| A | JP 4819657 B2 (TAIYO NIPPON SANSO CORP.) 24 November 2011 (2011-11-24)<br>See claims 1 and 2. | 1-9 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 September 2024** | **13 September 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.
**PCT/KR2024/008466**

**C. DOCUMENTS CONSIDERED TO BE RELEVANT**

| Category* | Citation of document, with indication, where appropriate, of the relevant passages | Relevant to claim No. |
|---|---|---|
| A | KR 10-2022-0017746 A (LG CHEM, LTD.) 14 February 2022 (2022-02-14) See claims 1-8 and figures 1 and 2. | 1-9 |

Form PCT/ISA/210 (second sheet) (July 2022)

**INTERNATIONAL SEARCH REPORT**
**Information on patent family members**

International application No.
**PCT/KR2024/008466**

| Patent document cited in search report | Publication date (day/month/year) | Patent family member(s) | Publication date (day/month/year) |
| --- | --- | --- | --- |
| KR 10-2022-0017749 A | 14 February 2022 | US 2023-0093912 A1 | 30 March 2023 |
| | | WO 2022-030940 A1 | 10 February 2022 |
| JP 2009-505055 A | 05 February 2009 | AU 2006-277712 A1 | 15 February 2007 |
| | | AU 2006-277712 B2 | 19 January 2012 |
| | | AU 2006-277713 A1 | 15 February 2007 |
| | | AU 2006-277713 B2 | 01 December 2011 |
| | | BR PI0614646 A2 | 12 April 2011 |
| | | BR PI0614746 A2 | 12 April 2011 |
| | | CA 2617511 A1 | 15 February 2007 |
| | | CA 2617513 A1 | 15 February 2007 |
| | | CN 101242783 A | 13 August 2008 |
| | | CN 101242783 B | 25 January 2012 |
| | | CN 101242783 C | 13 August 2008 |
| | | CN 101268155 A | 17 September 2008 |
| | | CN 101268155 B | 07 May 2014 |
| | | EP 1919361 A1 | 14 May 2008 |
| | | EP 1919361 B1 | 12 March 2014 |
| | | EP 1922367 A1 | 21 May 2008 |
| | | EP 1922367 B1 | 30 March 2016 |
| | | HK 1111072 A1 | 01 August 2008 |
| | | HK 1112934 A1 | 19 September 2008 |
| | | JP 2009-504833 A | 05 February 2009 |
| | | JP 4920685 B2 | 18 April 2012 |
| | | JP 5330828 B2 | 30 October 2013 |
| | | NZ 565662 A | 29 October 2010 |
| | | NZ 565663 A | 31 March 2011 |
| | | RU 2008-108486 A | 20 September 2009 |
| | | RU 2008-108607 A | 20 September 2009 |
| | | RU 2417239 C2 | 27 April 2011 |
| | | RU 2417749 C2 | 10 May 2011 |
| | | US 2007-0187587 A1 | 16 August 2007 |
| | | US 2007-0196656 A1 | 23 August 2007 |
| | | US 2012-0014858 A1 | 19 January 2012 |
| | | US 7923682 B2 | 12 April 2011 |
| | | US 8026328 B2 | 27 September 2011 |
| | | US 8530599 B2 | 10 September 2013 |
| | | WO 2007-017700 A1 | 15 February 2007 |
| | | WO 2007-017701 A1 | 15 February 2007 |
| | | ZA 200801118 B | 25 July 2012 |
| | | ZA 200801182 B | 30 July 2014 |
| JP 4819657 B2 | 24 November 2011 | JP 2008-128923 A | 05 June 2008 |
| KR 10-2022-0017746 A | 14 February 2022 | US 2023-0184727 A1 | 15 June 2023 |
| | | WO 2022-030941 A1 | 10 February 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020230078348